**Europäisches Patentamt**

**European Patent Office**

**Office européen des brevets**

(11) Veröffentlichungsnummer: **0 113 072**
**A2**

(12) **EUROPÄISCHE PATENTANMELDUNG**

(21) Anmeldenummer: **83112219.7**

(22) Anmeldetag: **05.12.83**

(51) Int. Cl.³: **G 01 N 33/54**

(30) Priorität: **06.12.82 US 446925**

(43) Veröffentlichungstag der Anmeldung:
**11.07.84 Patentblatt 84/28**

(84) Benannte Vertragsstaaten:
**AT BE CH DE FR GB IT LI NL SE**

(71) Anmelder: **F. HOFFMANN-LA ROCHE & CO. Aktiengesellschaft**
**CH-4002 Basel(CH)**

(72) Erfinder: **Giorgio, Nicholas A.**
**45 D Street**
**Avenel, N.J. 07001(US)**

(72) Erfinder: **Krupey, John**
**175 Rutland Road**
**Glen Rock, N.J. 07452(US)**

(72) Erfinder: **Wilson, Leonard T.**
**24 Sutton Place**
**Verona, N.J. 07044(US)**

(74) Vertreter: **Lederer, Franz, Dr. et al,**
**Patentanwälte Dr. Franz Lederer Reiner F. Meyer**
**Lucile-Grahn-Strasse 22**
**D-8000 München 80(DE)**

(54) Verfahren zur Bestimmung von CEA nach spezieller Probenvorbehandlung.

(57) Es wird ein verbessertes Verfahren zur Bestimmung des carzinoembryonischen Antigens (CEA) in einer menschlichen Serum- oder Plasmaprobe beschrieben. Im wesentlichen besteht die Verbesserung darin, dass die leicht sauer abgepufferte Serum- oder Plasmaprobe bei Raumtemperatur mit einem hydrophilen Kieselgel behandelt wird, worauf nach Abtrennung des Kieselgels in der Probe ein Immunverfahren zur Bestimmung des carzinoembryonischen Antigens durchgeführt wird.

EP 0 113 072 A2

F.HOFFMANN-LA ROCHE & CO. Aktiengesellschaft, Basel/Schweiz

0113072

_-1-_                          5. Dez. 1983

RAN 4060/126

## Verfahren zur Bestimmung von CEA nach spezieller Probenvorbehandlung

Die Bestimmung von carzinoembryonischem Antigen (im nachfolgenden CEA genannt) ist der Fachwelt bestens bekannt. Der Fachwelt ist ebenfalls bekannt, dass gewisse, nicht-spezifisch interferierende Substanzen, welche in der zu testenden Probe vorhanden sind, weitgehend entfernt oder neutralisiert werden müssen, damit eine exakte und empfindliche Bestimmung durchgeführt werden kann.

Es sind eine Reihe von Verfahren bekannt, nach denen möglicherweise interferierende Substanzen, die in der Probe der biologischen Flüssigkeit, z.B. in der Serum- oder Plasmaprobe, vorhanden sind, vor Bestimmung des CEA entfernt oder neutralisiert werden können. Es bedeutet klarerweise hinsichtlich Zeit, Kosten und Einfachheit der Durchführung des Tests einen Vorteil, die zur Entfernung solcher interferierenden Substanzen erforderlichen Handhabungen in einem gegebenen Test zu vereinfachen.

Bei der Bestimmung von CEA gemäss Freedman et al., (U.S.-Patent Nr. 3,663,684) wird die Blutprobe zuerst mit einem Glycoprotein-Lösungsmittel behandelt, indem CEA löslich ist, worauf die resultierende Lösung geklärt wird.

Klt/ 19.10.83

Beispiele solcher Lösungsmittel umfassen Perchlorsäure, Trichloressigsäure, Phosphorwolframsäure und dergleichen. Die Behandlung der Blutprobe mit dem Glycoprotein-Lösungsmittel erfolgt zum Zweck, die fällbaren Normalproteine und die interferierenden Antigenmaterialien zu entfernen. Das ausgefällte, interferierende Proteinmaterial wird darauf von der Probe durch Zentrifugation entfernt. Die Entfernung der Säure erfolgt entweder durch Dialyse oder Gelfiltration. Beide dieser Methoden sind zeitaufwendig, teuer und technisch lästig. Die Anzahl der Manipulationsschritte trägt ebenfalls zur Ungenauigkeit des Tests bei.

Die vorliegende Erfindung beschreibt ein Verfahren zur Vorbehandlung einer menschlichen Serum- oder Plasmaprobe für die anschliessende Bestimmung von CEA, welches rascher, einfacher und weniger aufwendig ist als die bekannten präparativen Methoden.

Wie bereits erwähnt beschreibt die vorliegende Erfindung ein Verfahren zur Herstellung von menschlichen Serumoder Plasmaproben für die anschliessende Bestimmung von CEA, welches rasch, bequem und weniger aufwendig ist als die vorbekannten Methoden. Das Verfahren gemäss vorliegender Erfindung umfasst die Verdünnung der Probe mit einem Puffer zu einem leicht sauren pH, das Mischen der Probe mit einem hydrophilen Kieselgel bei Raumtemperatur, das Stehenlassen des Gemisches während einer kurzen Zeit und die Abtrennung des Kieselgels von der Probe. Gegenstand der vorliegenden Erfindung ist auch ein verbessertes Verfahren zur Bestimmung von CEA, bei dem die hier beschriebene Vorbehandlung mitumfasst wird.

Das beim Verfahren gemäss vorliegender Erfindung verwendete hydrophile Kieselgel ist gekennzeichnet durch eine grosse spezifische Oberfläche und eine relativ kleine durchschnittliche primäre Partikelgrösse. Die spezifische Oberfläche des Kieselgels, das im Verfahren gemäss der vorliegenden Erfindung verwendet wird, ist von ungefähr 100

bis ungefähr 400 $m^2$/g und vorzugsweise von ungefähr 130 bis ungefähr 380 $m^2$/g. Die Oberfläche des Kieselgels wird nach der Methode von Brunauer et al. in J.A.C.S. 60, 309 (1958) bestimmt. Die durchschnittliche primäre Partikelgrösse des Kieselgels, das gemäss vorliegender Erfindung verwendet wird ist von ungefähr 7-15 Nanometer (Millimicron).

Die Menge an Kieselsäure, welche gemäss vorliegender Erfindung in der Praxis verwendet wird schwankt von 10 bis 100 mg und vorzugsweise von 35 bis 80 mg je Probe.

Die Probenvorbehandlungsmethode gemäss vorliegender Erfindung umfasst das Verdünnen der Probe von ungefähr 0,2 auf ungefähr 0,5 ml Plasma oder Serum.

Die Verdünnung der Testprobe mit einem sauren Puffer ist notwendig, um die Sensitivität der Bestimmung zu optimieren. Die so verdünnte Probe hat einen leicht sauren pH, d.h. einen pH von ungefähr 6 bis ungefähr 6,5 und vorzugsweise von 6,3. Beispiele von Puffern welche gemäss vorliegender Erfindung verwendet werden können umfassen herkömmliche saure Puffer, wie z.B. Ammoniumacetat-, Natriumacetat-, Natriumcitrat-Puffer und dergleichen. Es wird eine genügende Menge Puffer verwendet, um zu gewährleisten, dass der pH der Probe leicht sauer wird, d.h. einen pH von ungefähr 6 bis 6,5 aufweist.

Die Methode gemäss vorliegender Erfindung eliminiert die Perchlorsäureextraktion und die Notwendigkeit der Dialyse um die Probe von der Perchlorsäure zu reinigen. Die beachtlichen Zeit- und Kosteneinsparungen, welche durch die Vorbehandlungsmethode gemäss vorliegender Erfindung erzielt werden, machen die Bestimmung von CEA im grossen Masstab, z.B. für ein Massenscreening zugänglich. Ein vollständiges Verfahren zur Bestimmung von CEA unter Verwendung der Vorbehandlungsmethode gemäss vorliegender Erfindung kann in ungefähr 4 Stunden durchgeführt werden. Dies bedeutet eine wesentliche Verbesserung gegenüber käuflichen

CEA-Bestimmungen, bei denen über Nacht eine Dialyse durchgeführt werden muss.

In der Probe, die nach dem Verfahren gemäss vorliegender Erfindung vorbehandelt wurde, wird anschliessend eine geeignete CEA-Bestimmung durchgeführt. Die Wahl des jeweiligen Typs von Immunverfahren von CEA welches Verwendung finden soll, ist nicht kritisch für die Probenvorbehandlung gemäss vorliegender Erfindung. Im allgemeinen wird ein Radioimmunverfahren oder ein Enzymimmunverfahren bevorzugt, wobei jedoch ein Radioimmunverfahren besonders bevorzugt ist.

Im allgemeinen umfasst die Bestimmung von CEA gemäss vorliegender Erfindung die folgenden Schritte:

a) Vorbehandlung der Serum- oder Plasmaprobe eines Patienten gemäss vorliegender Erfindung zur Neutralisierung von möglicherweise interferierenden Materialien,

b) Zufügen eines Ueberschusses an CEA-Antikörpern zu den zu untersuchenden Proben und Inkubation während einer vorbestimmten Zeit,

c) Zufügen von markiertem CEA zu den Proben und Inkubation während einer vorgegebenen Zeit,

d) Zufügen eines Insolubilisierungsmittels zum Gemisch von b) und c) und Bildung einer Festphase enthaltend Antikörper-gebundenes CEA und eine flüssige Phase enthaltend ungebundenes CEA,

e) Trennen der festen und der flüssigen Phasen,

f) Bestimmen der Menge von Markierungsmittel entweder in der festen oder in der flüssigen Phase und

g) Bestimmen der Menge an CEA in der Probe durch Vergleich mit einem Standard.

Bei der oben erwähnten Bestimmungsmethode wird der Begriff "Neutralisieren" von möglicherweise interferierenden Materialien verwendet. Der Begriff "Neutralisieren" bezieht sich sowohl auf die erfindungsgemässe Probenvorbehandlung wie die vorbekannten Methoden, bei denen diese interferierenden Materialien physikalisch abgetrennt werden, da ja der gewünschte Effekt in beiden Fällen derselbe ist. Im Prinzip bedeutet also der Begriff "Neutralisieren", dass die interferierenden Materialien entfernt werden.

Wie früher erwähnt, kann das zur Markierung von CEA verwendete Markierungsmittel in der vorerwähnten Bestimmung jedes immunologisch verträgliche Markierungsmittel sein, welches eine quantitative Bestimmung zulässt, wie z.B. ein Radioisotop, ein Enzym, ein Fluoreszenz oder ein Chemilumineszenz oder dergleichen. Enzym- und Radioisotop-Markierungsmittel sind bevorzugt, wobei die letzteren besonders bevorzugt sind. Unter den Radioisotopen, welche herkömmlicherweise bei Radioimmunverfahren verwendet werden können, sind die Isotope von Jod bevorzugt, wobei Jod-125 besonders bevorzugt ist.

Das Insolubilisierungsmittel, welches verwendet wird, um eine feste Phase enthaltend Antigen-gebundenes CEA und eine flüssige Phase enthaltend ungebundenes CEA zu formen, kann jedes Material sein, welches für diesen Zweck bekannt ist. So z.B. können gewisse aliphatische Alkohole, Ionenaustauschharze und anorganische Salze sowie auch Antikörper, welche gegen den CEA-Antikörper gerichtet sind, die Bildung eines Proteinniederschlages bewirken, welcher Antigen-gebundenes CEA enthält. Ein bevorzugtes Insolubilisierungsmittel ist ein zweiter Antikörper, d.h. ein Antikörper, der gegen den CEA-Antikörper gerichtet ist, in immobilisierter Form, d.h. in insolubilisierter Form.

Bezüglich der vorerwähnten Immobilisierung des zweiten Antikörpers kann jede bekannte Technik zum Immobilisieren einer immunologischen Komponente, wie z.B. Partikel verschiedener Grösse, Kügelchen, Stäbchen oder Streifen eines Trägermaterials, verwendet werden. Sofern als Insolubilisierungsmittel ein Polymer verwendet wird, z.B. ein Styrolpolymer oder Copolymer, kann - entsprechend den individuellen Eigenschaften des Polymers - mehr als eine der oben erwähnten Formen verwendet werden. Ein besonders bevorzugtes Material zur Immobilisierung des zweiten Antikörpers ist ungesintertes Poly(vinylidenfluorid), welches z.B. in Form von feinen Partikeln oder als Film verwendet werden kann. Der Antikörper kann physikalisch an das immobilisierende Material gebunden sein oder nach konventionellen Methoden chemisch daran gebunden sein.

Die besonderen Verhältnisse, Inkubationszeiten und Temperaturen, welche für eine gegebene CEA-Bestimmung zu berücksichtigen sind, liegen im Hinblick auf die zahlreichen Publikationen betreffend CEA im Bereich des Fachwissens. Bevorzugte Inkubationszeiten für die Reaktion des Antiserums und die nachfolgende Reaktion mit dem markierten CEA sind von ungefähr 1 Stunde bis ungefähr 2 1/2 Stunden, wobei 2 Stunden besonders bevorzugt sind. Diese Inkubationen werden bei einer Temperatur von ungefähr 45°C durchgeführt. Sofern ein Enzymlabel verwendet wird, können Anpassungen der Inkubationszeit und besonders der Temperatur erforderlich sein, um die Desaktivierung eines Enzyms zu vermeiden, wenn das Enzym bei den vorstehend angegebenen Temperaturen labil ist. Sofern ein immobilisierter zweiter Antikörper als Insolubilisierungsmittel verwendet wird, werden die Proben vorzugsweise bei Umgebungstemperatur während ungefähr 10-30 Minuten, vorzugsweise während 15 Minuten inkubiert. Die Bestimmung von CEA in der Probe wird durch einen Vergleich mit einer Standardkurve in herkömmlicher Weise durchgeführt. Das Ablesen der Konzentration an Markierungsmittel wird abhängig vom Typ des Markierungsmittels ebenfalls nach herkömmlichen Methoden durchgeführt, z.B. unter Verwendung eines Gamma-Szintillations-Spektrometers, wenn ein Radionuclid-Markierungsmittel verwendet wird.

Die nachfolgenden Beispiele illustrieren die Erfindung weiterhin. Sofern nicht anders angegeben, sind alle Temperaturen in Celsiusgraden angegeben.

Beispiel 1

Die kommerzielle Bestimmung für CEA wird wie folgt durchgeführt:

Plasmaproben (0,5 ml; im Doppel) von Patienten mit Verdacht auf Colonkarzinom werden mit 2,5 ml kalter 1,2 molarer Perchlorsäure durch Mischen in einem Mischer vom Vortextyp während 30 Sekunden extrahiert und dann während 20 Minuten bei 1000 x g zentrifugiert. Die Ueberstände werden gesammelt und in einen Dialyseschlauch gegeben und dann gegen deionisiertes Wasser dialysiert, wobei das Wasser dreimal gewechselt wird und zwischen jedem Wechsel mindestens 3 Stunden gewartet wird. Es wird eine Schlussdialyse unter Verwendung eines Ammoniumacetatpuffers vom pH ungefähr 6,5 durchgeführt. Nach der Dialyse wird der Inhalt der Dialyseschläuche in Teströhrchen gegeben, worauf unter Mischen mit einem Vortexmischer 25 µl von kommerziell erhältlichem CEA-Antiserum zugegeben werden. Die Röhrchen werden während 30 Minuten bei 45° inkubiert. Zu jedem Röhrchen wird unter Mischen 25 µl $^{125}$J-CEA zugegeben, worauf die Röhrchen wiederum während 30 Minuten bei 45° inkubiert werden. Es werden 2,5 ml Zirconylphosphatgel (pH 6,25) zu jedem Röhrchen gegeben, worauf man während 5 Minuten bei 1000 x g zentrifugiert. Nach Entfernen der Ueberstände wird unter Mischen zu jedem Röhrchen 5 ml Ammoniumacetatpuffer vom pH 6,25 gegeben. Die Röhrchen werden erneut wie oben beschrieben zentrifugiert, und die Ueberstände werden entfernt. Der Anteil von gebundenem $^{125}$J-CEA im verbleibenden Gel wird während 1 Minute durch Zählen in einem Gamma-Szintillations-Spektrometer bestimmt.

Eine Standardinhibitionskurve wird wie folgt hergestellt:

Zu Paaren von Teströhrchen wird 5,0 ml einer 1:10-Verdünnung von EDTA-Puffer (pH 6,5) mit deionisiertem Wasser gegeben. In jedes Röhrchen wird eine CEA-Standarddosis, d.h. 0, 10, 25, 50 und 100 µl entsprechend 0, 2,5, 6,25, 12,5 und 25 ng/ml CEA-Aktivität gegeben, und die

Röhrcheninhalte werden mit einem Mischer vom Vortextyp gemischt. Die Röhrchen werden dann wie oben angegeben mit Antiserum zu CEA und $^{125}$J-CEA behandelt. Es wird abgelesen und eine Kurve erstellt, welche zum Vergleich der Resultate dient, die mit den Patientenplasmen enthalten wurde.

## Beispiel 2

Eine Bestimmung von CEA unter Verwendung der Vorbehandlung der Probe gemäss vorliegender Erfindung wie wie folgt durchgeführt:

Plasmaproben (0,5 ml) werden mit 2,9 ml eines 5,2mM Ammoniumacetatpuffers (pH 4) verdünnt und mit einem Mischer vom Vortextyp gemischt. Nach Zugabe von 1,7 ml einer 5%-igen wässrigen Suspension von Kieselgel (mit einer Oberfläche von ungefähr 380 m$^2$/g und einer durchschnittlichen primären Partikelgrösse von ungefähr 7 nm) wird gründlich gemischt bis die Probe homogen ist. Die Probe wird bei Umgebungstemperatur während ungefähr 10 Minuten inkubiert. Die Probe wird während 10 Minuten bei 1000 x g zentrifugiert, und der Ueberstand wird in ein anderes Röhrchen dekantiert. Zu jedem Röhrchen werden unter Mischen mit einem Mischer vom Vortextyp 25 µl von kommerziellen CEA-Antiserum zugegeben. Die Röhrchen werden während ungefähr 1 Stunde bei 45° inkubiert. Unter Mischen werden jedem Röhrchen 25 µl $^{125}$J-CEA zugegeben, worauf man während ungefähr 1,5 Stunden bei 45° inkubiert. Zu jedem Röhrchen wird 1 ml einer wässrigen Suspension eines Antikörpers gegen das CEA-Antiserum gegeben, welcher durch Adsorption an Partikel von Poly(vinylidenfluorid) insolubilisiert wurde. Die Röhrchen werden erneut gemischt und während ungefähr 15 Minuten bei Raumtemperatur inkubiert. Die Proben werden dann während 10 Minuten bei 1000 x g zentrifugiert. Die Ueberstände werden dekantiert, worauf die Radioaktivität des Rückstandes im Röhrchen in einem Gamma-Szintillations-Spektrometer bestimmt wird.

Eine Standardinhibitionskurve wird wie folgt hergestellt:

Zu Paaren von Teströhrchen werden 0,5 ml menschliches Plasma enthaltend 1 ng/ml CEA gegeben. Zu jedem Röhrchen wird eine CEA-Standarddosis, d.h. 0, 10, 25, 50 und 100 µl entsprechend 0, 2,5, 6,25, 12,5 und 25 ng/ml CEA-Aktivität gegeben, worauf man mit einem Mischer vom Vortextyp mischt. Die Röhrchen werden wie oben angegeben mit Ammoniumacetatpuffer, Kieselgel, Antiserum gegen CEA, $^{125}$J-CEA und insolubilisiertem zweiten Antikörper behandelt. Nach Ablesen wird eine Kurve erstellt, welche zum Vergleich der Resultate dient, welche mit den Plasmaproben des Patienten erhalten wurden.

In Tabelle I werden die Resultate für die Proben, welche nach der herkömmlichen Dialysemethode gemäss Beispiel 1 erhalten wurden, mit denjenigen Resultaten, die mit der erfindungsgemässen Methode gemäss Beispiel 2 erhalten wurden, verglichen.

## Tabelle I

Vergleich einer Standard-CEA-Bestimmung (Beispiel 1) und einer CEA-Bestimmung gemäss vorliegender Erfindung (Beispiel 2).

| Röhrchen | Beispiel 1 Standard-CEA-Bestimmung (CPM)* | %Bo** | Beispiel 2 Neue CEA-Bestimmung (CPM)* | %Bo** |
|---|---|---|---|---|
| Gesamt $^{125}$J-CEA | 123,100 | | 123,000 | |
| Nicht-spezifische Bindung | 3,600 | | 3,600 | |
| Bindung bei 0 CEA (Bo) | 87,800 | | 64,800 | |
| Bindung bei 2,5 ng/ml CEA | 78,600 | 89% | 60,000 | 93% |
| Bindung bei 6,25 ng/ml CEA | 60,000 | 68% | 45,800 | 70% |
| Bindung bei 12,5 ng/ml CEA | 42,200 | 48% | 31,900 | 49% |
| Bindung bei 25 ng/ml CEA | 26,700 | 50% | 24,600 | 38% |

CEA mittlere Kontrolle

    7,0 ng/ml         -------         7,8 ng/ml

CEA hohe Kontrolle

    14,6 ng/ml        -------       15,4 ng/ml

*(CPM)      = Counts pro Minute

**%Bo      = Prozent der Bindung von CEA zum Antikörper bei einer Null-Konzentration von Antigen

## Beispiel 3

Eine Bestimmung von CEA gemäss vorliegender Erfindung wird wie folgt durchgeführt:

Eine Plasmaprobe (0,2 ml) wird zu einem 13 x 100 ml Glasröhrchen gegeben. Zu dieser Probe werden 2,3 ml einer 1,85%-igen (g/V) Suspension von Kieselgel (Oberfläche ungefähr 380 $m^2$/g und durchschnittlicher primärer Partikeldurchmesser ungefähr 7 nm) in 3,3 mM Ammoniumacetatpuffer vom pH 4 gegeben. Die Probe wird gründlich gemischt bis die Probe homogen ist. Die Probe wird bei Umgebungstemperatur während ungefähr 15 Minuten inkubiert. Die Probe wird während 5 Minuten bei 1000 x g zentrifugiert, und die Ueberstände werden in ein anderes Röhrchen dekantiert. Zu jeder Probe werden unter Mischen mit einem Mischer vom Vortextyp 25 µl Ziegen-anti-CEA-Antiserum zugegeben. Die Röhrchen werden während 1 Stunde bei 45° inkubiert. Zu jedem Röhrchen wird unter Mischen 25 µl $^{125}$J-CEA zugegeben, worauf die Röhrchen erneut während ungefähr 1,5 Stunden bei 45° inkubiert werden. Zu jedem Röhrchen werden 0,6 ml einer wässrigen Suspension eines Antiköprers gegen das CEA-Antiserum gegeben, welcher durch Adsorption an Partikel von Poly(vinylidenfluorid) insolubilisiert wurde. Die Röhrchen werden erneut gemischt und während 15 Minuten bei Raumtemperatur inkubiert. Die Proben werden erneut während 10 Minuten bei 1000 x g zentrifugiert. Die Ueberstände werden dekantiert und der Röhrchenrückstand wird in einem Gamma-Szintillations-Spektrometer gemessen.

Eine Standardinhibitionskurve wird wie folgt erhalten:
Zu Paaren von Teströhrchen werden 0,2 ml menschliches
Plasma enthaltend weniger als 1 ng/ml CEA gegeben. Zu jedem
Paar von Röhrchen wird eine CEA-Standarddosis, d.h. 0, 10,
25, 50 und 100 µl entsprechend 0, 2,5, 6,25, 12,5 und
25 ng/ml von CEA-Aktivität zugegeben, worauf die Röhrcheninhalt mit einem Mischer vom Vortextyp gemischt werden.
Die Röhrchen werden wie oben beschrieben mit Antiserum
gegen CEA, $^{125}$J-CEA und insolubilisiertem zweiten Antikörper behandelt. Nach Ablesen der Resultate wird eine
Kurve erstellt, welche zum Vergleich der Resultate dient,
welche mit den Plasmaproben der Patienten erhalten wurden.

In Tabelle II werden die Resultate für die Proben gemäss herkömmlicher Dialysemethode nach Beispiel 1 und diejenigen gemäss Methode der vorliegenden Erfindung nach
Beispiel 3 verglichen.

## Tabelle II

Vergleich einer Standard-CEA-Bestimmung (Beispiel 1)
und einer CEA-Bestimmung nach vorliegender Erfindung
(Beispiel 3).

| Röhrchen | Beispiel 1 Standard-CEA-Bestimmung | | Beispiel 3 Neue CEA-Bestimmung | |
|---|---|---|---|---|
| | (CPM)* | %Bo** | (CPM)* | %Bo** |
| Total $^{125}$J-CEA | 123,000 | | 135,800 | |
| Nicht-spezifische Bindung | 3,600 | | 3,800 | |
| Bindung bei 0 CEA (Bo) | 87,800 | 100% | 86,000 | 100% |
| Bindung bei 2,5 ng/ml CEA | 78,600 | 89% | 80,000 | 93% |
| Bindung bei 6,25 ng/ml CEA | 60,000 | 68% | 65,900 | 77% |
| Bindung bei 12,5 ng/ml CEA | 42,200 | 48% | 52,500 | 61% |
| Bindung bei 25 ng/ml CEA | 26,700 | 30% | 37,000 | 43% |

CEA mittlere Kontrolle

     7,0 ng/ml         ------              6,3 ng/ml

CEA hohe Kontrolle

     14,6 ng/ml         ------           14,8 ng/ml

*(CPM)      = Counts pro Minute

**%Bo      = Prozent der Bindung von CEA zum Antikörper bei einer Antigenkonzentration von null.

- 14 -

Patentansprüche

1. Verfahren zur Vorbehandlung einer menschlichen Serum- oder Plasmaprobe für die anschliessende CEA-Bestimmung, welches die nachfolgenden Schritte umfasst:

(a) Zufügen von genügend Puffer, um die Probe auf einen pH von ungefähr 6 bis ungefähr 6,5 abzupuffern;

(b) Inkubieren der Probe in Gegenwart von hydrophilem Kieselgel, und

(c) Abtrennen des Kieselgels von der Probe.

2. Verfahren nach Anspruch 1, dadurch gekennzeichnet, dass das hydrophile Kieselgel eine Oberfläche von ungefähr 130 bis 380 $m^2$/g und eine durchschnittliche primäre Partikelgrösse von ungefähr 7-16 Nanometer aufweist.

3. Verfahren nach Anspruch 2, dadurch gekennzeichnet, dass das hydrophile Kieselgel eine Oberfläche von ungefähr 380 $m^2$/g und eine durchschnittliche primäre Partikelgrösse von ungefähr 7 nm aufweist.

4. Verfahren nach Anspruch 1, dadurch gekennzeichnet, dass als saurer Puffer ein Ammoniumacetatpuffer verwendet wird.

5. Verfahren nach Anspruch 1, dadurch gekennzeichnet, dass die Probe auf einen pH von ungefähr 6,3 abgepuffert wird.

6. Verfahren zur Bestimmung der Konzentration von carzinoembryonischem Antigen in einer menschlichen Serum- oder Plasmaprobe, welches die nachfolgenden Schritte umfasst:

a) Zufügen von genügend Puffer, um die Probe auf einen pH von ungefähr 6 bis ungefähr 6,5 abzupuffern,

b) Inkubieren der Probe in Gegenwart eines hydrophilen Kieselgels, um die Materialien in dieser Probe, welche mit der Bestimmung interferieren könnten, zu neutralisieren,

c) Abtrennen des Kieselgels von der Probe,

d) Zufügen vom Ueberschuss an Antikörper gegen CEA zur Probe und Inkubieren während einer vorbestimmten Zeit,

e) Zufügen einer Menge von carzinoembryonischem Antigen, welches mit einer Markierung versehen ist, die quantitativ bestimmt werden kann, die ausreicht, um mit der Menge von Antikörper der in Stufe d) hinzugefügt wurde, zu reagieren und Inkubieren während einer vorbestimmten Zeit,

f) Zufügen von Insolubilisierungsmittel zur Probe zur Bildung einer Festphase enthaltend Antikörper gebundenes CEA und eine flüssige Phase enthaltend ungebundenes CEA,

g) Trennen der festen und flüssigen Phasen,

h) Bestimmen der Menge der Markierungssubstanz entweder in der festen oder in der flüssigen Phase und

i) Bestimmen der Menge von carzinoembryonischem Antigen in der Probe durch Vergleich mit einem Standard.

7. Verfahren nach Anspruch 6, dadurch gekennzeichnet, dass das hydrophile Kieselgel eine Oberfläche von ungefähr 130 bis 380 $m^2$/g und eine durchschnittliche primäre Partikelgrösse von ungefähr 7-16 nm aufweist.

8. Verfahren nach Anspruch 7, dadurch gekennzeichnet, dass das hydrophile Kieselgel eine Oberfläche von ungefähr 380 $m^2$/g und eine durchschnittliche primäre Partikelgrösse von ungefähr 7 nm aufweist.

9. Verfahren nach Anspruch 6, dadurch gekennzeichnet, dass als saurer Puffer ein Ammoniumacetatpuffer verwendet wird.

10. Verfahren nach Anspruch 6, dadurch gekennzeichnet, dass die Probe auf ungefähr pH 6,3 abgepuffert wird.

11. Verfahren nach Anspruch 6, dadurch gekennzeichnet, dass in Stufe d) als Insolubilisierungsmittel ein Antikörper verwendet wird, wobei dieser Antikörper in insolubilisierter Form ist.

12. Verfahren nach Anspruch 6, dadurch gekennzeichnet, dass die Markierungssubstanz ein Radioisotop ist.

13. Verfahren nach Anspruch 12, dadurch gekennzeichnet, dass das Radioisotop ein Radioisotop von Jod ist.

14. Verfahren nach Anspruch 13, dadurch gekennzeichnet, dass das Radioisotop von Jod Jod-125 ist.

15. Verfahren nach Anspruch 6, dadurch gekennzeichnet, dass als Markierungssubstanz ein Enzym verwendet wird.

16. Verfahren nach Anspruch 6, dadurch gekennzeichnet, dass als Markierungssubstanz ein fluoreszierendes Mittel verwendet wird.

17. Verfahren nach Anspruch 6, dadurch gekennzeichnet, dass als Markierungssubstanz ein chemilumineszierendes Mittel verwendet wird.

**